# EUROPEAN PATENT APPLICATION

(11) **EP 0 995 757 A2**
(43) Date of publication of application: **26.04.2000**
(21) Application number: 99116689.3
(22) Date of filing: 26.08.1999
(51) Int. Cl.: C07K 5/062, A61K 47/48, A61K 38/05

(54) **Bivalent inhibitors of the proteasome**

(30) Priority: 26.08.1998 EP 98116127
(71) Applicant: Max-Planck-Gesellschaft zur Förderung der Wissenschaften e.V., 80539 München (DE)
(72) Inventor: Ditzel, Lars, 36142 Tann 1, a.d. Rhön (DE); Groll, Michael, 94072 Bad Füssing (DE); Huber, Robert, Prof. Dr., 82110 Germering (DE); Loidl, Günther, 93333 Neustadt/Donau (DE); Musiol, Hans-Jürgen, 81475 München (DE); Moroder, Luis, Prof. Dr., 82152 Martinsried (DE)
(74) Representative: Huber, Bernhard, Dipl.-Chem.

(57) **Abstract**

The invention comprises a bivalent proteasome inhibitor compound comprising two head groups and a spacer linking the two head groups and, wherein the two head groups can be the same or different and are each capable of inhibiting one or more active sites of the proteasome and wherein the spacer comprises a flexible, substantially linear organic compound with a length of 25 Å to 100 Å.

## Description

The invention comprises new proteasome inhibitors that are based on the use of polyethyleneglycol (PEG) as a flexible and linear spacer of defined length to crosslink two head groups capable of interacting with one or more active sites of the multienzyme complex and thus to produce bivalent inhibitors of high affinity and selectivity.

Proteasomes are ubiquitous in all three kingdoms of life. The proteasome is a multicatalytic protease complex that is responsible for the intracellular protein turnover in procaryotes and eucaryotes. As it is involved in many pathophysiological processes like inflammation and cancer, it constitutes a promising target for drug design.

The X-ray structure of the 20S proteasome from the archaebacterium *Thermoplasma acidophilum* at 3.4 Å resolution showed that it is a particle of cylindrical shape in which the 28 subunits are arranged as four homo-heptameric rings α₇β₇β₇α₇ (Löwe, J. et al., Science 268, 533-539, 1995). The N-terminal threonine residue of the β-subunits was found to bind via hemiacetal formation to inhibitory peptide aldehydes, so it was concluded that this residue is involved in the hydrolytic activity (Löwe, J. et al. Science 268, 533-539, 1995; Seemüller, E. et al. Science 268, 579-582, 1995), relating the proteasome to the Ntn ('N-terminal nucleophile') family of hydrolases (Brannigan, J. et al., Nature 378, 416-419, 1995).

The yeast (*Saccharomyces cerevisiae*) 20S proteasome is similar in size and shape to the archaebacterial complex, but it is much more complex with 7 different α-type and 7 different β-type subunits, all of which have been cloned and sequenced and can be grouped by sequence homology (Heinemeyer, W. et at., Biochem. 33, 12229-12237, 1994). As defined by the character of the P1 cleavage sites of chromogenic substrates, trypsin-like, chymotrypsin-like, and peptidylglutamyl-peptide hydrolytic (PGPH) activities are exhibited by the eukaryotic proteasome. These specificities, however, are not reflected in the cleavage pattern of protein substrates, which can be cleaved at almost every position (Ehring, B. et al., Eur. J. Biochem. 235, 404-415, 1996; Dick, L.R. et al., Immunol. 152, 3884-3894, 1994; Wenzel, T. et al., FEBS Lett. 349, 205-209, 1994; Kuckelkorn, U. et al., Eur. Immunol. 25, 2605-2611, 1995).

The crystal structure of the 20S proteasome from the yeast *Saccharomyces cerevisiae* showed that its 28 protein subunits are arranged as an (α1-α7, β1-β7)₂ complex in four stacked rings (Groll, M. et al., Nature 386, 463-471, 1997). The primary strucures of these 14 subunits have been discovered by cloning and sequencing techniques. The β-type proteins are encoded by the PRE1, PRE2, PRE3, PRE4, PRS3, PUP1 and PUP3 genes, whereas the α-type proteins are encoded by the genes PRS1, PRS2, Y7, Y13, PUP2, PRE5 and PRE6. The active sites are again located in the interior of the particle which is apparently accessible only by some very narrow side entries.

Three of the seven different β-type subunits are processed at the N-terminal nucleophile, the threonine-1 residue, which is essential for activity. The S1-pockets of three β-subunits are the major specificity determinants and are appropiately polar and sized to accommodate acidic, basic and apolar P1 side chains, respectively, but also bind non-complementary residues. They are responsible for the three major proteolytic activities of the yeast proteasome and display inhibitor binding sites suggesting that the β1/PRE3 has post-glutamylpeptide hydrolytic (PGPH) specificity, the β2/PUP1 has trypsin-like activity and that the β5/PRE2 subunit has chymotrypsin-like specificity.

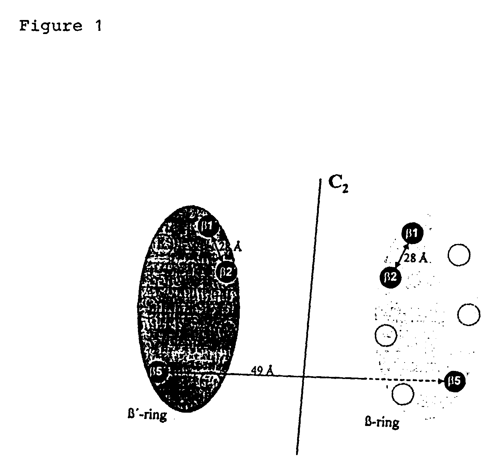

The crystal structure of the 20S proteasome from *Saccharomyces cerevisiae* (Groll, M. et al., Nature 386, 463-471, 1997) revealed a symmetrical arrangement of the three active sites with their PGPH, trypsin- and chymotrypsin-like specifities at the level of each of the two central β-rings in the cavity of the cylindrical particle as schematically outlined in Figure 1, providing a schematic representation of the active sites and their intra- and inter-ring distances: β1 and β1' (PGPH), β2 and β2' (trypsin-like activity), β5 and β5' (chymotrypsin-like activity). Well defined intra- and inter-ring distances between the different active sites can be derived from this crystal structure.

**Table 1**

| The Oγ→Oγ distances between two Thr-1 residues of the different active sites of the proteasome from *S. cerevisiae* as derived from the coordinates of the X-ray structure (Groll, M. et al., Nature 386, 463-471, 1997). | |
|---|---|
| **intra-ring distances** | **inter-ring distances** |
| β1 β2 27,95 Å | β1 β1' 29,16 Å |
| β1 β5 64,94 Å | β2 β2' 64,99 Å |
| β2 β5 64,38 Å | β5 β5' 49,44 Å |
| | β1 β2' 50,45 Å |
| | β1 β5' 60,60 Å |
| | β2 β5' 42,86 Å |

The eukaryotic 20S proteasomes, including those from yeast, are very closely related in function, amino acid sequences of their components, and in the structure as shown by electronmicroscopy. The α-type and β-type subunits of the mammalian 20S proteasome are arranged in an ordered and unique way (Kopp, F. et al., J. Mol. Biol. 248, 264-272, 1995). In mammalian cells, three additional non-essential subunits of the 20S proteasome LMP2, LMP7, and MECL1 can replace constitutive components upon induction by the T-cell derived antiviral cytokine interferon-γ. The LMP subunits are encoded in a region of the MHC gene cluster adjacent to the TAP (transporters associated with antigen-presentation) genes, which are involved in transport of antigenic peptides from the cytosol to the endoplasmic reticulum. Their expression or targeted deletion alters the proteasome's peptidase specificity and MHC class I cell-surface expression level (Gaczynska, M. et al., Nature 365, 264-267, 1993; Driscoll, J. et al., Nature 365, 262-264, 1993; Fehling, H.J. et al., Science 265, 1234-1237, 1994; Van Kaer, L. et al., Immunity 1, 533-541, 1994). The properties of peptide fragments generated by the proteasome correlate strongly with those of MHC class I ligands and processing by proteasomes is probably the major pathway of MHC class I peptide generation (Goettrup, M. et al., Immunol. Today 17, 429-435, 1996).

The main biological functions of the proteasome in both the cytosol and the nucleus are removal of abnormal, misfolded or improperly assembled proteins, stress response (by processing or degradation of transcriptional regulators), cell cycle control (by degradation of cyclins), cell differentiation and metabolic adaptation (by destruction of transcription factors or metabolic enzymes), and the cellular immune response. Proteasomes are responsible for the generation of most antigenic peptides as ligands for MHC class I molecules by degrading non-self antigens with a relatively defined octapeptide pattern for presentation by the MHC proteins to the immunecompetent cells. Therefore they are a target for modifying or silencing antigen processing and presentation to T cells, which are important in transplant rejection and autoimmune diseases. Silencing a single active site can modulate antigen presentation without eliminating essential housekeeping functions (Groettrup M. and Schmidtke G., DDT, Vol 4, No 2, 1999, 63-71).

All those cellular functions mentioned above are linked to an ubiquitin and ATP requiring protein degradation pathway involving the 26S proteasome whose core and proteolytic chamber is formed by the 20S proteasome. Its essential nature is reflected in the lethality of chromosomal deletions of all but one (α3/Y13) (Emori, Y. et al., Mol. Cell. Biol. 11, 344-353, 1991) of its subunits in yeast (Rivet, A.J., Biochem J. 291, 1-10, 1993; Peters, J.-M., TIBS 19, 377-382, 1994; Goldberg, A.L. et al., Chem. & Biol. 2, 503-508, 1995; Hochstrasser, M., Curr. Op. Cell. Blot. 7, 215-223, 1995; Rubin, D.M. and Finley, D., Curr. Biol. 5, 854-858, 1995; Lord, M., Curr. Biol. 6, 1067-1069, 1996; Coux, O. et al., Ann. Rev. Biochem. 65, 801-847, 1996; Stock D. et al., J. Curr. Op. Biotech. 7, 376-385, 1996, Stock et al., J. Co. Spr. Harb. Symp. on Quant. Biol. LX, 525-532, 1995; Chen, P. and Hochstrasser, M., EMBO J. 14, 2620-2630, 1995; Groettrup, M. et al., Immunol. Today 17, 429-435, 1996; Hilt, W. and Wolf, D.H., TIBS 21, 96-102, 1996).

Once the proteasome was thought to be responsible only for the catabolism of abnormal proteins, now it is known that the degradation pathway by the proteasome plays a key role in the turnover and processing of many regulatory proteins (Rivett, A.J., Biochem. J. 291, 1-10, 1993; Peters, J.-M. TIBS 19, 377-382, 1994; Goldberg, A.L. et al., Chem. & Biol. 2, 503-508, 1995; Hochstrasser, M., Curr. Op. Cell. Biol. 7, 215-223, 1995; Rubin, D.M. and Finley, D., Curr. Biol. 5, 854-858, 1995; Lord, M., Curr. Biol. 6, 1067-1069, 1996; Coux, O. et al., Ann. Rev. Biochem. 65, 801-847, 1996). Several of these proteolytic processes represent key biochemical events that contribute to the pathogenesis of human diseases. Thus, the proteasome is now being recognized as an important molecular target for drug discovery by developing specific and potent inhibitors (Adams, J. and Stein, R., Ann. Rep. Med. Chem. 31, 279-288, 1998). Proteasome inhibitors are also potential drugs in the treatment of malaria.

In the past, there have been several approaches to designing inhibitors of the proteasome. The Ntn character of the active sites of the proteasome is very similar to the active sites of serine and cysteine proteases and thus, the knowledge accumulated in the past for developing reversible and irreversible inhibitors of serine and cysteine proteases has been applied for the design of proteasome inhibitors.

The proteasome exhibits a low degree of substrate specificity due to its multiple functions in the cell. Therefore the design of specific inhibitors of the proteasome proves to be exceedingly difficult.

Among the irreversible inhibitors reported for the proteasome like 3,4-dichloroisocoumarin (Peireira, M.E. et al., J. Biol. Chem. 267, 7949-7955, 1992; Cardoza, C. et al., Biochem. 31, 7373-7380, 1992), peptidyl-α'β'-epoxyketones (Spaltenstein, A. et al., Tetrahedron Lett. 37, 1343-1346, 1996), peptidyl-vinylsulfones (Bogoyo, M. et al., Proc. Natl. Acad. Sci. USA 90, 6629-6634, 1997; Bogyo, M. et al., Chem. & Biol. 5, 307-320, 1998), peptidyl-sulfonyl-fluorides (Levine, L., Cell. Signalling 10, 653-659, 1998), peptidyl-chloromethyl and -diazomethyl-ketones (Reidlinger, J. et al., J. Biol. Chem. 272, 24899-24095, 1997) and lactacystin (Fenteany, G. et al., Science 268, 726-731, 1995), only lactacystin shows a high degree of selectivity.

Since irreversible inhibitors are of limited therapeutical interest, great attention has been paid in recent years to the design and synthesis of monovalent reversible inhibitors by focusing mainly on the use of peptide aldehydes, peptide boronic acids and their esters, which are described in detail in Adams, J. and Stein, R. *Annu. Reports in Med. Chem.* **31,** 279-288 (1998), peptide ketoaldehydes (Lynas, J.F. et al., Bioorg. Med. Chem. Lett. 8, 373-378, 1998), 5-methoxy-1-indanone dipeptide benzamines (Lum, R.T. et al., Bioorg. Med. Chem. Lett. 8, 209-214, 1998) and ketocarbonyl derivatives (Iqbal, M. et al., Bioorg. Med. Chem. Lett. 6, 287-290, 1996).

The binding mode of the peptide aldehydes is based on hemiacetal formation between the Thr1Oγ of the active site and the carbonyl carbon of the aldehyde as well assessed by X-ray analysis of proteasome/peptide aldehyde adducts (Löwe, J. et al., Science 268, 533-539, 1995; Groll, M. et al., Nature 386, 463-471, 1997; Escherich, A. et al., Biol. Chem. 378, 893-898, 1997; Loidl, G. et al., 25th Eur. Peptide Symp. Budapest, 30.8.-6.9.1998, Abstr.).

WO 95/25533 discloses tripeptide aldehydes which are derivatized at their N-terminus in various ways and which contain natural and non-natural amino acid residues with mainly hydrophobic side chains at positions P1 and P3. These tripeptide aldehydes are used for the inhibiton of chymotryptic activity and are examples of the monovalent inhibitors described in reference 20.

Similar tripeptide aldehyde inhibitors are also disclosed in WO 95/24914. Those compounds are again monovalent tripeptide aldehydes with hydrophobic P1 residues.

WO 96/13266 also relates to tripeptide-like structures containing C-terminal boronic ester and acid groups. Those inhibitors are monovalent and belong to the type reviewed in reference 20.

X-ray analyses of the proteasome/inhibitor complexes further confirm the difficulty in designing specific inhibitors as they clearly revealed that peptide aldehydes of structures designed for inhibiting the chymotrypsin-like β5 and β5' active sites are found in all six active sites, i.e. even in the trypsin-like β2 and β2' as well as in the PGPH β1 and β1' active sites. Independently of the C-terminal anchor group mainly large size hydrophobic side chains have been exploited in position P1, P2 and P3 to develop highly potent inhibitors of the chymotryptic activity of the proteasome as well described in WO 95/25533 and Escherich, A. *et al.,* L. *Biol. Chem.* **378,** 893-898 (1997). Thereby, cross-inhibition of cathepsin B and calpain represents a major obstacle for therapeutical applications.

Multivalent inhibitors of certain molecules have been produced using various spacer molecules. Among these are polyethylene glycol and poly-α-amino acids. WO96/11213 describes bivalent inhibitors of the soluble cytokine interleukin 4 (IL-4) which consist of two IL-4-receptor moieties linked together by a polyethylene glycol (PEG) spacer.

Another problem with known inhibitors of the proteasome is the fact that the proteasome has its active centres inside the complex. Therefore, the entering proteins must possess a certain structure. The proteins are ubiquitinated and denatured and then enter the proteasome. The entry mechanism has not been elucidated but it is known that peptides must be in a linear conformation (cyclic peptides cannot enter the proteasome). This means that the kinetics of inhibitor binding will be very different from those of soluble molecules with equally accessible active sites.

Thermodynamically the binding of multivalent molecules to different binding sites is similar to the chelate effect (Crothers, D.M. and Metzger, H., Immunochem. 9, 341-357, 1972; Nen, D. et al., J. Mol. Biol. 246, 367-373, 1995; Spike, C.G. and Parry, R. W., J. Am. Chem. Soc. 75, 2726-2729, 1953). The common feature of these binding events is that following the initial recognition and binding or reaction at one active site, each succeeding event, i.e. recognition and binding at a second active site, is more favorable because the entropy loss is decreased. Thus, exploiting these beneficial entropic effects of multivalency on avidity, it may be possible to increase binding affinities by several orders of magnitude, if inhibitors are available containing two active site binding moieties spaced by a properly designed molecule.

For the design of the bivalent inhibitors capable of spanning distances of 50 Å or more, the main difficulty is the right choice of the spacer. Taking into account the ubiquitin-proteasome pathway of protein degradation and the information derived from the X-ray structure of the proteasome, for a protein to be hydrolyzed by the proteasome, it must first be completely unwound and cystine bridges must be reduced and the linear polypeptide chain is then threaded through a narrow passage into the inner channel of the proteasome for proteolysis. Correspondingly, bulky scaffolds can not be used for oligo-presentation of the inhibitors.

Peptides of the appropriate length cannot be used as spacers because they are natural targets. Peptides of this size like gastrin (17mer) or secretin (27mer) were found to be rapidly degraded, fully confirming previous studies on the degradation of peptides by the proteasome (Wenzel, T. et al., FEBS Lett. 349, 205-209, 1994). Furthermore, it was found that binding of inhibitors from the S'-sites to the Thr1 residue is highly unfavoured. This will cause problems when octapeptide spacers are used.

Another disadvantage of spacers of the state of the art is that they are often too hydrophobic and therefore are likely to cause hydrophobic collapses that would prevent the molecule to be threaded into the cylinder in an extended unfolded form.

Therefore, it is an object of the present invention to provide compounds that are suitable as inhibitors of the proteasome and which exhibit increased binding affinities in comparison with monovalent inhibitors.

This object defined above is achieved according to the invention by providing bivalent proteasome inhibitor compounds comprising two head groups and a spacer linking the two head groups, wherein the two head groups can be the same or different and are each capable of inhibiting one or more of the active sites of the proteasome and wherein the spacer comprises a flexible, substantially linear organic compound with a length of from 25 Å to 100 Å.

As mentioned above and shown in Table 1, the intra-ring and inter-ring distances between different active sites of the proteasome span between around 25 Å and 70 Å. The present invention describes the design and synthesis of bivalent inhibitors based on these specific distances. A suitable spacer should therefore have a length which allows the two head groups linked thereto to interact with the two active centres of the proteasome which are to be inhibited by the bivalent inhibitor of the invention.

Thus the preferred length of spacer is from about 25 Å to 100 Å, preferably from about 28 Å to about 65 Å. More preferably, the spacer group spans a distance of from about 50 Å to about 65 Å.

The preferred minimum spacer lengths for each individual pair of active sites to be inhibited by the bivalent inhibitor of the invention can be seen in Table 1.

It was also found to be preferable to use a spacer which allows docking of the inhibitory head groups from the non-primed site of the substrate binding clefts of the β1, β2, β5 and β1', β2', β5' subunits, respectively.

Suitable compounds that can be used as spacers for the inhibitors of the invention are flexible, substantially linear organic molecules, examples of which are linear peptides comprised of D-amino acids, oligomers of β and/or γ-amino acids, of β, γ and/or ε sugar amino acids, hydroxy-substituted carboxylic acids, linear polysaccharides, and molecules consisting of several oxyalkylene units such as polyethylene glycol (PEG), as well as derivatives of these compounds.

Surprisingly, it was found that polyethylene glycol (PEG) and derivatives thereof are suitable as a spacer for bivalent inhibitors of the proteasome. Polyethylene glycol is a fully water-soluble and fully unstructured linear polymer that mimics perfectly unstructured polypeptide chains. PEG is hydrophilic so that hydrophobic collapses can be avoided.

It was found that by linking two monovalent proteasome inhibitors of the state of the art, their potency can be markedly increased (see Table 3).

Therefore, a preferred bivalent inhibitor of the invention comprises two head groups and a spacer linking the two head groups, wherein the two head groups can be the same or different and are each capable of inhibiting one or more of the active sites of the proteasome and wherein the spacer comprises polyethylene glycol comprising 4 to 50 oxyethylene units or a derivative thereof.

For the purposes of the invention it is preferred to use a PEG derivative with 4 to 50, more preferably 10 to 40, more preferably 18 to 25, still more preferably 19 to 25 oxyethylene units. In principle, any monovalent compounds that have shown inhibitory activity against the proteasome can be used as head groups to produce the bivalent inhibitors of the present invention. The inhibitors of the invention may comprise two identical head groups (homobivalent inhibitors) or two different head groups (heterobivalent inhibitors).

Especially preferred are bivalent inhibitor compounds of the general formula I:

**X-CH(R1)-NH-[P]**_{**n**}**-Y-Z-Y'-[P']**_{**m**}**-NH-CH(R1')-X'** (general formula I)

wherein the various groups in this formula can represent the following:
X-CH(R1)-NH-[P]ₙ and [P']ₘ-NH-CH(R1')-X' are identical (homovalent inhibitors) or different (heterobivalent inhibitors) and wherein X and X' are groups capable of interacting in reversible and irreversible mode with the Thr1 residue of the proteasome and are groups selected from aldehyde, boronic acid, boronic acid ester, α-ketoaldehyde, α-ketoester, α-ketoamide, trifluoromethyl ketone, diazomethane for reversible inhibition, and/or from isocoumarin derivatives, α',β'-epoxyketone, vinyl-sulfone, chloromethyl and diazomethylketone, sulfonyl-fluoride for irreversible inhibition.
R1 and R1' are identical or different and correspond to the specificity of the S1 enzyme subsite for chymotrypsin- or trypsin-like or PGPH activity of the proteasome exhibited by the subunits β5/β5', β2/β2' and β1/β1', respectively. Any two groups that allow a reversible or irreversible inhibition of the proteasome can, in principle, be employed. R1 and R1' are preferably selected from hydrophobic side chains of L or D natural and unnatural amino acids and linear or branched alkyl, cycloalkyl-, alkaryl, and aralkyl residues which may be amino-, guanido-, amidino- or carboxy-substituted.
For inhibition of β2 and β2' preferred residues are the side chains of arginine, lysine, ornithine or citrulline or related mimetic structures e.g. saturated or unsaturated, branched or unbranched alkyl or cycloalkyl, alkylaryl, aralkyl residues which may be amino-, guanido-, amidino-substituted.
For the inhibition of β1 and β1', all the side chains of glutamic and aspartic acid and all residues that mimic such side chains, e.g. carboxy-alkyl, carboxy-aryl, carboxy-alkylaryl or carboxy-aralkyl residues are suitable and preferred.
For inhibition of β5 and β5', all hydrobic side chains of natural amino acids, e.g. of leucine, norleucine, valine, tryptophan, phenylalanine or residues that mimic such side chains, e.g. branched or unbranched, saturated or unsaturated alkyl, cycloalkyl, aralalkyl, alkylaryl, aryl, biphenyl, condensed benzene rings (naphtyl), aromatic and non aromatic heterocyclic groups, etc can be used.
Preferred hydrophobic side chains within the definition of R1 and R1' are linear or branched alkyl, cycloalkyl, alkenyl, aryl and -CH₂R2 where R2 is aryl, aralkyl, alkaryl, cycloalkyl or -Q-R3 where Q is a chalcogen, and R3 is alkyl.
P and P' are identical or different and correspond to the P2 to P7 substrate positions and are natural or unnatural L- or D-amino acids with hydrophobic or hydrophilic or charged side chains. The side chain of P2 is pointing to the bulk solvent according to the X-ray structure of the proteasome/inhibitor complexes (Löwe, J. et al., Science 268, 533-539, 1995; Groll, M. et al. Nature 386, 463-471, 1997; Escherich, A. et al., Biol. Chem. 378, 893-898, 1997; Loidl, G. et al., 25th Eur. Peptide Sym. Budapest, 30.8.-6.9.1998, Abstr. and Proc. Natl. Acad. Sci. 96, 5418-5422,1999) and does not contribute to the binding affinity; the P3 residue contains a side chain that for the inhibition of β5 and β5' is a large hydrophobic side chain that can be branched or unbranched saturated or unsaturated alkyl, aryl, arylalkyl, aromatic and non-aromatic heterocyclic structure (e.g. indole, naphtyl) etc. and for β2 and β2' similar to the P3 residue for β5 and β5' or Arg, Lys etc. and related mimetic structures.
m and n are identical or different and are 0, 1, 2, 3, 4, 5 or 6.
Y and Y' are identical or different and correspond to the formula CO-(A)-(B) where (A) is an alkyl, alkenyl, cycloalkyl, alkaryl, aralkyl or heteroaromatic group and (B) is CO or NH or omitted.
Z is O-CH₂-CH₂-(O-CH₂-CH₂)ₙ-O, OC-CH₂-(O-CH₂-CH₂)ₙ₋₁-O-CH₂-CO or NH-CH₂-CH₂-(O-CH₂-CH₂)ₙ-NH with n varying from 4 to 50.

In the general formula I alkyl and alkenyl residues per se or within aralkyl or alkaryl residues may contain from 1 to 20 resp. 2 to 20 C-atoms, preferably 1 to 12 resp. 2 to 12 C-atoms, especially 1 to 8 resp. 2 to 8 C-atoms if bound to an aryl residue.

As head groups it is therefore possible to use any reactive handles known from the state of the art of serine and cysteine protease inhibitors. Preferred head groups are [NH-CO-(CH₂)₂-CO-Leu-Leu-Nle-H], [NH-CO-(CH₂)₂-CO-Arg-Val-Arg-H] and combinations thereof.

Most preferred inhibitor compounds of the present invention are the compounds (PEG)₁₈₋₂₅-, preferably (PEG)₁₉₋₂₅-(NH-CO-(CH₂)₂-CO-Leu-Leu-Nle-H]₂ (**4**) and (PEG)₁₉₋₂₅-[NH-CO-(CH₂)₂-CO-Arg-Val-Arg-H]₂ (**6**) as homobivalent inhibitors and (PEG)₁₉₋₂₅-[NH-CO-(CH₂)₂-CO-Arg-Val-Arg-H][NH-CO-(CH₂)₂-CO-Leu-Leu-Nle-H] (**7**) as heterobivalent inhibitor, as shown in Table 3.

Another aspect of the present invention is a method for preparing the bivalent proteasome inhibitor compounds of the invention comprising linking two monovalent proteasome inhibitor molecules capable of inhibiting one or more active sites of the proteasome with a spacer molecule comprising a flexible, substantially linear organic compound with a length of 25 Å to 100 Å, especially with the terminal amino or carboxy groups of polyoxyethylene or a derivative thereof comprising 4 to 50 oxyethylene units.

Preferred head groups to be used are those defined above. Preferable spacers are also the same as those mentioned above.

Another aspect of the invention relates to a pharmaceutical composition for the inhibition of proteasome function comprising a bivalent inhibitor compound of the invention or a physiologically acceptable salt thereof as effective compound, and optionally physiologically carriers, additives, thinners and/or other auxiliary substances.

In another aspect, the present invention also concerns the uses of the bivalent inhibitor compounds of the invention. Since they are suitable for inhibiting the proteasome they can be used for a number of purposes.

Via inhibition of the proteasome, the overall rate of intracellular protein degradation can be reduced. This may be of particlular use in cells, which degrade muscle proteins too fast or in an uncontrollable fashion.

The bivalent inhibitor compounds can also be used to inhibit the activity of NF-KappaB in a cell. Proteasome inhibitors prevent activation of NF-κB by stabilizing the phosphorylated form of I-κB. Since NF-κB drives transcription of numerous pro-inflammatory cytokines including TNFα, IL-1, IL-2, IL-6 and IL-8, inhibition of pro-inflammatory mediators can be achieved by inhibiting the proteasome with the inhibitors described above and thus, suppressing NF-κB gene expression. Thereby, the NF-κB dependent cell adhesion can also be inhibited.

This provides a powerful approach to blocking the inflammatory cascade. Moreover the results of a delayed type hypersensitivity assay in mice make proteasome inhibitors promising for the treatment of allergic skin reactions and an arthritis model in rats showed the potentials of proteasome inhibitors for treatment of chronic inflammation (Adams, J. and Stein, R., Ann. Rep. Med. Chem. 31, 279-288, 1998; Palombella, V. et al., Proc. Natl. Acad. Sci. USA 95, 15672-15676, 1998; Lee, D.H. and Goldberg, A.L., Trends in Cell Biology 8, 397-403, 1998).

Thus, inhibition of proteasome using the bivalent inhibitors of the invention can inhibit cyclin degradation, degradation of p53 protein and also help to prevent growth of cancer cells.

Further, the bivalent inhibitors of the invention are useful as anti-inflammatory agents, agents to inhibit antigen presentation and can help to prevent the replication of viruses, especially HIV.

The following examples are intended to illustrate some embodiments without limiting the scope of the invention.

### Example 1

### Proteasome assay

A solution of proteasome from *Saccharomyces cerivisiae* in Tris-buffer (pH 7.5; 450 µl; 6.67 nM for PGPH, 5.56 nM for trypsin-like and 1.11 nM for chymotrypsin-like activity) was incubated with the inhibitors at varying concentrations (1 nmol to 100 µmol) for 1 h at 37°C. The fluorogenic substrates for PGPH (Z-Leu-Leu-Glu-βNA, 40 µM), trypsin-like (Bz-Phe-Val-Arg-AMC, 8 µM) and chymotrypsin-like assays (Suc-Leu-Leu-Val-Tyr-AMC, 8 µM) were dissolved in the same Tris-buffer with a minimum amount of DMSO and added to the enzyme solution at 37 °C to reach a final volume of 500 µl. Fluorescence excitation/emission wavelengths were 360 nm/460 nm for AMC and 335 nm/410 nm for βNA. The rates of hydrolysis were monitored by the fluorescence increase and the initial linear portions of curves (100-300 sec) were used to calculate the IC₅₀ values.

### Example 2

### X-Ray Structure Analysis

Crystals of 205 proteasomes from *S. cerivisiae* were grown in hanging drops at 24 °C as described previously (Groll et al., *Nature* **386,** 463-471, 1997). The protein concentration used for crystallization was 40 mg/ml in 10 mM Tris-HCl (pH 7.5) and 1 mM EDTA. The drops contained 4 µl protein and 2 µl reservoir solution with 30 mM magnesium acetate, 100 mM morpholino-ethane-sulphonic acid (pH 6.5) and 11 % MPD. The inhibitors were soaked with a final concentration of 5 mM for 12h. Data were collected using synchrotron radiation with λ = 1.1 Å on the BW6-beamline at the DESY-Centre/Hamburg/Germany (Table 1). Crystals were soaked in a cryoprotecting buffer (30% MPD, 28 mM magnesium acetate, 100 mM morpholino-ethane-sulfonic acid (pH 6.9) and frozen in a stream of cold nitrogen gas at 90 K (Oxford Cryo Systems). X-ray intensities were evaluated by using the MOSFILM program package (version 5.3) and data reduction was performed with CCP4. The anisotropy of diffraction was corrected by an overall anisotropic temperature factor by comparing observed and calculated structure amplitudes using X-PLOR (Brunger, A. (1992) X-PLOR: A System for X-Ray Crystallography and NMR, version 3.1; Yale University Press, New Haven, CT). Electron density was averaged 10 times over the 2-fold non crystallographic symmetry axis using MAIN (Turk, D., Ph. D. Thesis, Technical University, Munich, 1992). Model building was carried out with FRODO (Jones, T.A., J. Appl. Cryst. 11, 268-272, 1978). Modelling experiments were performed using the coordinates of yeast 20S proteasome with MAIN (Turk, D., Ph.D. Thesis, Technical University, Munich, 1992).

**Table 2**

| **Data collection and refinement statistics** | | |
|---|---|---|
| 20S proteasome complex with | compound **4** | compound **6** |
| Space group | P2₁ | P2₁ |
| Crystal data (Å/°) | a=135.4 | a=134.3 |
| | b=298.9 | b=300.7 |
| | c=144.6 | c=143.9 |
| | | β=113.0 |
| | β=112.9 | |
| Resolution (Å) | 2.3 | 3.0 |
| Observation (>2σ) | 1224818 | 389546 |
| Unique reflections | 437435 | 175637 |
| Completeness (%) | 94.4 | 86.4 |
| R_{merge} (%) | 9.7 | 17.3 |
| R/R_{free} (%) | 25.3/30.0 | 20.7/28.5 |
| rms from bonds (Å) | 0.011 | 0.012 |
| rms from angles (°) | 1.875 | 1.966 |

### Eample 3

**Table 3**

| ***Inhibition of yeast proteasome by mono- and bivalent inhibitors** **(IC***_{***50***}***, µM).*** | | | |
|---|---|---|---|
| **Inhibitor** | **β1/β1'** | **β2/β2'** | **β5/β5'** |
| Ac-LLnL-H (**1**) | >100 | >100 | 2.1 |
| Ac-RVR-H (**2**) | >100 | 6.4 | >100 |
| HOOC-(CH₂)₂-CO-NH-(PEG)₁₉₋₂₅-NH-(CH₂)₂-CO-LLnL-H (**3**) | >100 | >100 | 1.8 |
| (PEG)₁₉₋₂₅-[NH-CO-(CH₂)₂-CO-LLnL-H]₂ (**4**) | >100 | >100 | 0.017 |
| HOOC-(CH₂)₂-CO-NH-(PEG)₁₉₋₂₅-NH-(CH₂)₂-CO-RVR-H (**5**) | >100 | 8.2 | >100 |
| (PEG)₁₉₋₂₅-[NH-CO-(CH₂)₂-CO-RVR-H]₂ (**6**) | >100 | 0.071 | >100 |
| (PEG)₁₉₋₂₅-[NH-CO-(CH₂)₂-CO-RVR-H][NH-CO-(CH₂)₂-CO-LLnL-H] (**7**) | >100 | 0.097 | 0.031 |

The inhibition potencies of the homobivalent compounds **4** and **6** of the general formula I for inhibition of the chymotryptic activity (β5/β5') and tryptic activity (β2/β2') of the proteasome in comparison with the related monovalent inhibitors **1** and **2**, respectively, are shown in Table 3.

Taking into account the distances between the β5/β5' and β2/β2' active sites of ca 50 Å and 65 Å as well as the need of sufficient flexibility for docking of the binding headgroups from the S-subsites to two active sites commercial diamino-PEG with a statistical length distribution of 19 to 25 monomers and with the terminal amino groups capped with succinic acid was used to crosslink two molecules of leucyl-leucyl-norleucinal (H-Leu-Leu-Nle-H) and arginyl-valyl-arginal (H-Arg-Val-Arg-H) for the production of the homobivalent inhibitors **4** and **6**. To analyze the effect of the PEG moiety on binding affinities of the tripeptide aldehydes **1** and **2**, the related monopegylated derivatives **3** and **5** were prepared.

Similarly, with compound **7** a heterobivalent inhibitor containing a binding headgroup for the trypsin-like active sites β2 and β2' and a binding headgroup for the chymotrypsin-like active sites β5 and β5' was synthesized. A comparison of the inhibitory potency of the pegylated tripeptide aldehydes **3** and **5** with those of the parent compounds **1** and **2** shows practically no effect of the PEG moiety (Table 3).

Conversely, the homobivalent inhibitors **4** and **6** exhibit a 100-fold increased activity. In the case of heterobivalent inhibitors, one molecule can neutralize only one active site of the existing pair. Two molecules are thus required for complete inhibition, however, with the advantage of inhibiting two activities concomitantly. As expected, with the heterobivalent inhibitor **7** both the trypsin- and chymotrypsin-like activities are inhibited (Table 3). The inhibitory potencies against the trypsin- and chymotrypsin-like activities are similar to those of the homobivalent inhibitors, if the stoichiometry of this type of inhibitor is taken into account.

As expected from the results obtained with the proteasome/Ac-Leu-Leu-Nle-H complex (Groll et al., *Nature* **386,** 463-471, 1997), in the X-ray structure of the proteasome/**4** complex even the homobivalent inhibitor **4** occupies all six active sites with the inhibitory head groups binding in identical mode to the active sites as the parent tripeptide aldehyde. Since the inhibition data support bivalent binding, occupation of all six active sites in absence of competing substrates is expected to occur both in the intra- and inter-ring mode, since the spacer length is sufficient for all possible bridgings within their maximal distances of approximately 65 Å. Moreover, the size of the spacer allows for binding of the inhibitory headgroups from the S subsites in all six subunits. The electron-density map does not reveal a conformationally restricted PEG moiety in any part of the cavity.

In the case of the homobivalent inhibitor **6** of the trypsin-like activity, the X-ray analysis of the complex revealed the presence of the inhibitory headgroup Arg-Val-Arg-H only in the two β2 and β2' active sites. This result indicates that affinity of a basic P1 residue is highly restricted to the S1 subsite of the trypsin-like activity. Again the PEG spacer is not detectable and probably fluctuates freely in the cavity of the proteolytic chamber.

As shown in Table 3 and confirmed by X-ray analysis, the bivalent inhibitors **4, 6** and **7** corresponding to the invention showed an increase in binding affinity of about two orders of magnitude if compared to the monovalent inhibitors **1, 2, 3** and **5**.

As theoretically expected, the bivalent inhibitor **4** shows an increase in binding affinity of about two orders of magnitude if compared to inhibitor **1** or to the monovalent inhibitor **3** (Table 3).

In the case of bivalent inhibitors inhibitory moieties that show *per se* weak affinity can be used as head groups and enhancement of affinity is obtained by the bivalency. Thus, moieties which as monovalent inhibitors are poorly inhibiting other serine or cysteine proteases such as calpain or cathepsin B can be used to obtain potent proteasome inhibitors of correspondingly high specificity.

### Example 4

### Synthesis of the inhibitors

### H-Leu-Leu-Nle-Sc trifluoroacetate

The title compound was prepared as described previously (Schaschke, N. et al. FEBS Lett. 391, 297-301, 1996).

### Ac-Leu-Leu-Nle-H (1)

The compound was obtained by acetylation of H-Leu-Leu-Nle-Sc trifluoroacetate with acetic anhydride followed by hydrolysis of the semicarbazone derivative (Escherich, A. et al. Biol. Chem. 378, 893-898, 1997).

### H-Val-Arg(Adoc)₂-DEA

The title compound was prepared as described previously (Loidl, G. et al., Chem. Biol. 6, 197-204, 1999).

### Z-Arg(Adoc)₂-Val-Arg(Adoc)₂-DEA

Z-Arg(Adoc)₂-OH (0.301 g; 0.45 mmol), TBTU (0.145 g; 0.45 mmol), HOBt (0.062 g; 0.45 mmol) and DIEA (0.156 ml; 0.90 mmol) were dissolved in 2 ml DMF, and at 0 °C a solution of H-Val-Arg(Adoc)₂-DEA (0.311 g; 0.45 mmol) in 2 ml DMF was added. After stirring overnight at room temperature the solvent was removed and the residue distributed between AcOEt and 5% KHSO₄. The organic layer was washed with 5% KHSO₄, 5% NaHCO₃ and water, and dried over MgSO₄. The solvent was removed and the oily residue redestilled with toluene; yield: 0.565 g (93%) of a white foam; TLC (CHCl₃/ACN/AcOH; 20:1:0.1): R_{f} 0.6.

### H-Arg(Adoc)₂-Val-Arg(Adoc)₂-DEA

Z-Arg(Adoc)₂-Val-Arg(Adoc)₂-DEA (0.530 g; 0.40 mmol) was hydrogenated in 100 ml of 95% EtOH over Pd/C (10%). After 1 h the catalyst was filtered off and the filtrate evaporated to give a colorless foam; yield: 0.468 g (98%); TLC (CHCl₃/MeOH/AcOH; 20:1:0.1): R_{f} 0.6; HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 13.36 min.

### Ac-Arg(Adoc)₂-Val-Arg(Adoc)₂-DEA

To a solution of H-Arg(Adoc)₂-Val-Arg(Adoc)₂-DEA (180 mg; 0.15 mmol) in 10 ml DMF was added acetic anhydride (0.142 ml; 1.5 mmol) and DIEA (0.256 ml; 1.5 mmol). After 1 h the solvent was removed under reduced pressure and the residue distributed between AcOEt and 5% NaHCO₃. The organic layer was washed with 5% KHSO4 and H₂O, dried over MgSO₄ and evaporated to yield a white foam; yield: 180 mg (97%); TLC (CHCl₃/MeOH; 20:1): R_{f} 0.6; HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 14.63 min.

### Ac-Arg-Val-Arg-H × 2TFA (2)

Deprotection of Ac-Arg(Adoc)₂-Val-Arg(Adoc)₂-DEA (41 mg; 0.033 mmol) was carried out by reaction with 1 ml of 95% TFA at 0°C. After 90 min the product was precipitated with cold diethyl ether; yield: 18 mg (80%); TLC (AcOEt/BuOH/AcOH/H₂O; 2:1:1:1): R_{f} 0.3; ES-MS: *m/z* = 456.4 [M+H]⁺; calcd. Mᵣ = 455.30.

### HOOC-(CH₂)₂-CO-NH-(PEG)₁₉₋₂₅-NH-(CH₂)₂-CO-Leu-Leu-Nle-Sc

The succinyl-amino-(PEG)₁₉₋₂₅ (Rapp Polymere, Tübingen, Germany) (100 mg, 88 µmol), Leu-Leu-Nle-Sc trifluoroacetate (Schaschke, N. et al. FEBS Lett. 391, 297-301, 1996) (45 mg, 88 µmol), TBTU (28 mg, 88 µmol), HOBt (12 mg, 88 µmol) and DIEA (45 µl, 264 µmol) were dissolved in 6 ml DMF. After 3 h the solvent was evaporated and the oily residue was distributed between methylene chloride and 5% KHSO₄. The aqueous phase was extracted twice with methylene chloride and the combined organic layers were washed with 5% NaHCO₃, dried over Na₂SO₄ and evaporated *in vacuo* to a colorless oil; yield: 90 mg (68%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min] Rₜ = 7.70 min. The same syntheses in this and the following experiments were also done with staring material containing (PEG)₁₈₋₂₅.

### (PEG)₁₉₋₂₅-[NH-CO- (CH₂)₂-CO-Leu-Leu-Nle-Sc]₂

The succinyl-amino-(PEG)₁₉₋₂₆ (Rapp Polymere, Tübingen, Germany) (100 mg, 88µmol), Leu-Leu-Nle-Sc trifluoroacetate (Schaschke, N. et al. FEBS Lett. 391, 297-301, 1996) (90 mg, 176 µmol), TBTU (57 mg, 176 µmol), HOBt (24 mg, 176 µmol) and DIEA (90 µl, 528 µmol) were dissolved in 1 ml DMF. After 4h the solvent was evaporated and the oily residue was purified by preparative HPLC on Nucleosil 250/C18 (Macherey & Nagel, Düren, Germany) with a linear gradient of acetonitrile/water from 15:85 to 60:40 in 70 min. Fractions containing homogeneous material as monitored by HPLC were combined and lyophilized; yield: 80 mg of a colorless oil (49%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 8.73 min.

### HOOC-(CH₂)₂-CO-NH-(PEG)₁₉₋₂₅-NH-(CH₂)₂-CO-Leu-Leu-Nle-H (3)

HOOC-(CH₂)₂-CO-NH-(PEG)₁₉₋₂₅-NH-(CH₂)₂-CO-Leu-Leu-Nle-Sc (80 mg, 53 µmol) was dissolved in a mixture of 5 ml MeOH, 2 ml acetic acid and 2 ml formaldehyde (37 % in water). After 2 h the solvent was removed and the oily residue was immediately purified by preparative HPLC on Nucleosil 250/C18 (Macherey & Nagel, Düren, Germany) with a linear gradient of acetonitrile/water from 15:85 to 60:40 in 70 min. Fractions containing homogeneous material as monitored by HPLC were combined and lyophilized; yield of colorless oil: 8 mg (10%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 7.99 min; ES-MS: *m/z* = 1508.6 ± (44.0)ₙ with n = 0 to 3 [M+H]⁺; calcd. Mᵣ = 1507.91 ± (44.03)ₙ with n = 0 to 3.

### (PEG)₁₉₋₂₅-[NH- CO- (CH₂)₂- CO-Leu-Leu-Nle-H]₂ (4)

(PEG)₁₉₋₂₅-[NH-CO-(CH₂)₂-CO-Leu-Leu-Nle-Sc]₂ (80 mg, 53 µmol) was dissolved in a mixture of 5 ml MeOH, 2 ml acetic acid and 2 ml formaldehyde (37 % in H₂O). After 2 h the solvent was removed and the oily residue was immediately purified by preparative HPLC on Nucleosil 250/C18 (Macherey & Nagel, Düren, Germany) with a linear gradient of acetonitrile/water from 15:85 to 60:40 in 70 min. Fractions containing homogeneous material as monitored by HPLC were combined and lyophilized; yield of colorless oil: 35 mg (20%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 9.56 min; ES-MS: *m/z* = 1832.6 ± (44.0)ₙ with n = 0 to 3 [M+H]⁺; calcd. Mᵣ = 1831.17 ± (44.03)ₙ with n = 0 to 3.

### HOOC-(CH₂)₂-CO-NH-(PEG)₁₉₋₂₅-NH-CO-(CH₂)₂-CO-Arg(Adoc)₂-Val-Arg(Adoc)₂-DEA

A mixture of the succinyl-amino-(PEG)₁₉₋₂₅ (200 mg, 0.18 mmol), H-Arg(Adoc)₂-Val-Arg(Adoc)₂-DEA (232 mg, 0.19 mmol), TBTU (62 mg, 0.19 mmol), HOBt (26 mg, 0.19 mmol) and DIEA (96 µl, 0.56 mmol) was stirred at room temperature for 3 h. After removal of the solvent the oily residue was distributed between methylene chloride and 5% KHSO₄. The aqueous phase was extracted twice with methylene chloride and the combined organic layers were washed with 5% NaHCO₃, dried over Na₂SO₄ and evaporated *in vacuo* to a colorless oil. The crude material was purified by size exclusion chromatography on Fractogel TSK HW 40 S (Merck AG, Darmstadt, Germany) using MeOH as eluent. Fractions containing homogeneous material as monitored by HPLC were pooled and evaporated; yield of colorless oil: 142 mg (35%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 12.3 min; ES-MS: *m/z* = 2368.6 ± (44.0)ₙ with n = 0 to 3 [M+H]⁺; calcd. Mᵣ = 2368.42 ± (44.03)ₙ with n = 0 to 3.

### (PEG)₁₉₋₂₅-[NH-CO-(CH₂)₂-CO-Arg(Adoc)₂-Val-Arg(Adoc)₂-DEA]₂

To a solution of HOOC-(CH₂)₂-CO-NH-(PEG)₁₉₋₂₅-NH-CO-(CH₂)₂-CO-Arg(Adoc)₂-Val-Arg(Adoc)₂-DEA (43 mg; 18 µmol), TBTU (12 mg, 37 µmol), HOBt (5 mg, 37 µmol) and DIEA (6.3 µl, 37 µmol) in 1 ml DMF was added H-Arg(Adoc)₂-Val-Arg(Adoc)₂-DEA (44.5 mg, 37 µmol) in 1 ml DMF. After stirring at room temperature for 2 h the sovent was removed and the oily residue distributed between methylene chloride and 5% KHSO₄. The organic layers were washed with 5% NaHCO₃, dried over Na₂SO₄ and evaporated *in vacuo* to a colorless oil. The crude material was purified by size exclusion chromatography on Fractogel TSK HW 40 S (Merck AG, Darmstadt, Germany) using MeOH as eluent. Fractions containing homogeneous material as monitored by HPLC were pooled and evaporated; yield of colorless foam: 44 mg (68%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 14.60 min; ES-MS: *m/z* = 1775.6 ± (22.0)ₙ with n = 0 to 3 [M+2H]²⁺; calcd. Mᵣ = 3552.18 ± (44.03)ₙ with n = 0 to 3.

### (PEG)₁₉₋₂₅-[NH-CO-(CH₂)₂-CO-Arg(Adoc)₂-Val-Arg(Adoc)₂-DEA][NH-CO-(CH₂)₂-CO-Leu-Leu-Nle-Sc]

HOOC-(CH₂)₂-CO-NH-(PEG)₁₉₋₂₅-NH-CO-(CH₂)₂-CO-Arg(Adoc)₂-Val-Arg(Adoc)₂-DEA (54 mg, 23 µmol), Leu-Leu-Nle-Sc trifluoroacetate (12 mg, 23 µmol), TBTU (7.4 mg, 23 µmol), HOBt (3.1 mg, 23 µmol) and DIEA (3.9 µl, 23 µmol) were reacted in 3 ml DMF for 3 h. After removal of the solvent the oily residue was distributed between methylene chloride and 5% KHSO₄. The aqueous phase was extracted twice with methylene chloride and the combined organic layers were washed with 5% NaHCO₃, dried over Na₂SO₄ and evaporated *in vacuo*; yield of colorless oil: 58 mg (92%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 13.3 min; ES-MS: *m/z* = 2748.3 ± (44.0)ₙ with n = 0 to 3 [M+H]⁺; calcd. Mᵣ = 2748.71 ± (44.03)ₙ with n = 0 to 3.

### HOOC-(CH₂)₂-CO-NH-(PEG)₁₉₋₂₅-NH-CO-(CH₂)₂-CO-Arg-Val-Arg-H × 2TFA (5)

*HOOC-(CH*_{*2*}*)*_{*2*}*-CO-NH-(PEG)*_{*19-25*}*-NH-CO-(CH*_{*2*}*)*_{*2*}*-CO-Arg(Adoc)*_{*2*}*-Val-Arg(Adoc)*_{*2*}*-DEA* (31 mg, 13 µmol) was reacted with 95% TFA for 2 h. Then the solvent was evaporated and the oily residue purified by preparative HPLC on Nucleosil 250/C18 (Macherey & Nagel, Düren, Germany). Fractions containing homogeneous material as monitored by HPLC were combined and lyophilized; yield of colorless oil: 19 mg (81 %); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 7.95 min; ES-MS: *m/z* = 1582.0 ± (44.0)ₙ with n = 0 to 3 [M+H]⁺; calcd. Mᵣ = 1579.93 ± (44.03)ₙ with n = 0 to 3.

### (PEG)₁₉₋₂₅-[NH-CO-(CH₂)₂-CO-Arg- Val-Arg-H]₂ × 4TFA (6)

(PEG)₁₉₋₂₅-[NH-CO-(CH₂)₂-CO-Arg(Adoc)₂-Val-Arg(Adoc)₂-DEA]₂ was deprotected by reaction with 95% TFA. After 2 h the solvent was removed and the oily residue distributed between H₂O and diethyl ether. The aqueous phase was evaporated under reduced pressure and the residue was lyophilized; yield of white powder: 15 mg (88%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 7.36 min; ES-MS: *m/z* = 989.2 ± (22.0)ₙ with n = 0 to 3 [M+2H]²⁺; calcd. Mᵣ = 1975.21 ± (44.03)ₙ with n = 0 to 3.

### (PEG)₁₉₋₂₅-[NH-CO-(CH₂)₂-CO-Arg-Val-Arg-H][NH-CO-(CH₂)₂-CO-Leu-Leu-Nle-H] (7)

(PEG)₁₉₋₂₅-[NH-CO-(CH₂)₂-CO-Arg(Adoc)₂-Val-Arg(Adoc)₂-DEA][NH-CO-(CH₂)₂-CO-Leu-Leu-Nle-Sc] (52 mg, 19 µmol) was dissolved in a mixture of 0.5 ml MeOH, 0.2 ml acetic acid and 0.2 ml formaldehyde (37 % in water). After 2 h the mixture was distributed between methylene chloride and 5% KHSO₄. The aqueous phase was extracted twice with methylene chloride and the combined organic layers were washed with 5% NaHCO₃, dried over Na₂SO₄ and evaporated *in vacuo*. The residue was reacted with 95% TFA for 2 h and after evoparation of the solvent the crude product was immediately purified by preparative HPLC on Nucleosil 250/C18 (Macherey & Nagel, Düren, Germany). Fractions containing homogeneous material as monitored by HPLC were combined and lyophilized; yield of white powder: 19 mg (47%); HPLC [column: Nucleosil 300/C8 (Macherey & Nagel, Düren, Germany), linear gradient of acetonitrile/2% phosphoric acid from 5:95 to 80:20 in 12 min with two additional isocratic minutes at a flow rate of 1 ml/min]: Rₜ = 9.03 min; ES-MS: *m/z* = 952.4 ± (22.0)ₙ with n = 0 to 3 [M+2H]²⁺; calcd. Mᵣ = 1903.19 ± (44.03)ₙ with n = 0 to 3.

### References

1. Emori, Y., Tsukahara, T., Kawasaki, H., Ishiura, S., Sugita, H. and Suzuki, K. *Mol. Cell. Biol*. **11**, 344-353 (1991).
2. Rivett, A.J. *Biochem J*. **291**, 1-10 (1993); Peters, J.-M. *TIBS* **19**, 377-382 (1994); Goldberg, A.L., Stein, R. and Adams, J. *Chemistry & Biology* **2**, 503-508 (1995); Hochstrasser, M. *Curr. Opin. in Cell Biol.* **7,** 215-223 (1995); Rubin, D.M. and Finley, D. *Current Biology* **5**, 854-858 (1995); Lord, M. *Current Biology* **6**, 1067-1069 (1996); Coux, O., Tanaka, K. and Goldberg, A.L. *Annu. Rev. Biochem.* **65**, 801-847 (1996).
2a. Stock, D., Nederlof, P.M., Seemüller, E., Baumeister, W., Huber, R. and Löwe, J. *Curr. Opin. Biotech*. **7**, 376-385 (1996); Stock, D., Ditzel, L., Baumeister, W., Huber, R. and Löwe, J. *Cold Spring Harbor Symposia on Quantitative Biology* **LX**, 525-532 (1995).
3. Chen, P. and Hochstrasser, M. *EMBO J*. **14**, 2620-2630 (1995).
4. Groettrup, M., Soza, A., Kuckelkorn, U. and Kloetzel, P.M. *Immunol. Today* **17,** 429-435 (1996).
5. Hilt, W. and Wolf, D.H. *TIBS* **21**, 96-102 (1996).
5a. Hilt, W., Enenkel, C., Gruhler, A., Singer, T. and Wolf, D.H. *J.Biol.Chem.* **268**, 3479-3486 (1993).
6. Löwe, J., Stock, D., Jap, B., Zwickl, P., Baumeister, W. and Huber, R. *Science* **268,** 533-539 (1995).
7. Seemüller, E., Lupas, A., Stock, D., Löwe, J., Huber, R. and Baumeister, W. *Science* **268,** 579-582 (1995).
8. Brannigan, J. A., Dodson, G., Duggleby, H.J., Moody, P.C.E., Smith, J.L., Tomchick, D.R. and Murzin, A.G. *Nature* **378,** 416-419 (1995).
9. Heinemeyer, W., Tröndle, N., Albrecht, G.and Wolf, D.H. *Biochemistry* **33,** 12229-12237 (1994).
10. Ehring, B., Meyer, T.H., Eckerskorn, C., Lottspeich, F. and Tampé, R. *Eur.J.Biochem*. **235,** 404-415 (1996).
11. Dick, L.R., Aldrich, C., Jameson, S.C., Moomaw, C.R., Pramanik, B.C., Doyle, C.K., DeMartino, G.N., Bevan, M.J., Forman, J.M. and Slaughter, C.A. *Immunol.* **152,** 3884-3894 (1994).
12. Wenzel, T., Eckerskorn, C., Lottspeich, F. and Baumeister, W. *FEBS Lett.* **349,** 205-209 (1994).
13. Kuckelkorn, U., Frentzel, S., Kraft, R., Kostka, S., Groettrup, M. and Kloetzel, P.-M. *Eur.J.Immunol.* **25,** 2605-2611 (1995).
14. Grall, M., Ditzel, L., Löwe, J., Stock, D., Bochtler, M., Bartunik, H.D. and Huber, R. *Nature* **386,** 463-471 (1997).
15. Kopp, F., Kristensen, P., Hendil, K.B., Johnson, A., Sobek, A. and Dahlmann, B. *J. Mol. Biol*. **248,** 264-272 (1995).
16. Gaczynska, M., Rock, K.L. and Goldberg, A.L. *Nature* **365,** 264-267 (1993).
17. Driscoll, J., Brown, M.G., Finley, D. & Monaco, J.J. *Nature* **365,** 262-264 (1993).
18. Fehling, H.J., Swat, W., Laplace, C., Kühn, R., Rajewsky, K., Müller, U. and von Boehmer, H. *Science* **265,** 1234-1237 (1994).
19. Van Kaer, L., Ashton-Rickardt, P.G., Eichelberger, H., Gaczynska, M., Nagashima, K., Rock, K.L., Goldberg, A.L., Doherty, P.C. and Tonegawa, S. *Immunity* **1**, 533-541 (1994).
20. Adams, J. and Stein, R. *Annu. Reports in Med. Chem.* **31,** 279-288 (1998).
21. Pereira, M.E., Nguyen, T., Wagner, B.J., Margolis, J.W., Yu, B., and Wilk, S. *J. Biol. Chem.* **267,** 7949-7955 (1992).
22. Cardoza, C., Vinitsky, A., Hidalgo, M.C. and Orlowski, M. *Biochemistry* **31**, 7373-7380 (1992).
23. Spaltenstein, A., Leban, J.J., Huang, J.J., Reinhardt, K.R., Viveros, O.H., Sigafoos and J., Crouch, R. *Tetrahedron Lett.* **37**, 1343-1346 (1996).
24. Bogyo, M., McMaster, J.S., Gaczynska, M., Tortorella, D., Goldberg, A.L. and Ploegh, H. *Proc. Natl. Acad. Sci. USA* **90**, 6629-6634 (1997).
25. Bogyo, M., Shin, S., McMaster, J.S. and Ploegh, H. *Chemistry & Biology* **5,** 307-320 (1998).
26. Reidlinger, J., Pike, A.M., Savory, P.J., Murray, R.Z. and Rivett, A.J. *J. Biol. Chem.* **272,** 24899-24095 (1997).
27. Fenteany, G., Standaert, R.F., Lane, W.S., Choi, S., Corey, E.J. and Schreiber, S.L. *Science* **268,** 726-731 (1995).
28. Lynas, J.F., Harriot, P., Healy, A., McKervey, M.A. and Walker, B. *Bioorg. Med. Chem. Lett.* **8**, 373-378 (1998).
29. Iqbal, M., Chatterjee, S., Kauer, J.C., Mallamo, J.P., Messina, P.A., Reiboldt, A. and Siman, R. *Bioorg. Med. Chem. Lett.* **6**, 287-290 (1996).
30. Escherich, A., Ditzel, L., Musiol, H.-J., Groll, M., Huber, R. and Moroder, L. *Biol. Chem.* **378,** 893-898 (1997).
31. Loidl, G., Musiol, H.J., Groll, M., Ditzel, L., Huber, R. and Moroder, L. *25*^{*th*} *Eur. Peptide Symp*. Budapest, 30.8.-6.9.1998, Abstr.
32. Crothers, D.M. and Metzger, H., *Immunochemistry* **9**, 341-357 (1972).
33. Nen, D., Momo, M., Prospero, T. and Winter, G. *J. Mol. Biol.* **246,** 367-373 (1995).
34. Spike, C.G. and Parry, R.W. *J. Am. Chem. Soc.* **75**, 2726-2729 (1953).
35. Schaschke, N., Musiol, H.-J., Assfalg-Machleidt, I., Machleidt, W., Rudolph-Böhner, S. and Moroder, L. *FEBS Lett*. **391,** 297-301 (1996).
36. Levine, L., *Cellular Signalling* **10,** 635-659 (1998)
37. Lum, R.T., Nelson, M.G., Joly, A.G., Lee, G., Meyer, S.M., Wick, M.M. and Schow, S.R., *Bioorg. Med. Chem. Lett.* **8**, 209-214 (1998)
38. Loidl, G., Groll, M., Musiol, H.-J., Ditzel, L., Huber, R. and Moroder, L., *Chem. Biol.* **6**, 197-204, (1999)
39. Palombella, V.J., Conner, E.M., Fuseler, J.W., Destree, A., Davis, J.M. Laroux, F.S., Wolf, R.E., Huang, J., Brand, S., Elliott, P.J., Lazarus, D., Mc Cormack, T., Parent, L., Stein, R., Adams, J., Gisham, M.B., *Proc. Natl. Acad. Sci USA* **95**, 15672-15676, (1998)
40. Lee, D.H. and Goldberg, A.L., *Trends in Cell Biology* **8**, 397-403, (1998)
41. Turk, D., *Ph. D. Thesis*, Technical University, Munich, (1992)
42. Jones, T.A., *J. Appl. Cryst.* **11**, 268-272, (1978)

## Claims

1. A bivalent proteasome inhibitor compound comprising two head groups and a spacer linking the two head groups, wherein the two head groups can be the same or different and are each capable of inhibiting one or more active sites of the proteasome and wherein the spacer comprises a flexible, substantially linear organic compound with a length of 25 Å to 100 Å.

2. The bivalent proteasome inhibitor compound of claim 1 wherein the spacer comprises polyethylene glycol comprising 4 to 50 oxyethylene units or a derivative thereof.

3. The bivalent proteasome inhibitor compound according to claim 1 oder 2,
wherein it is of the general formula I:
X-CH(R1)-NH-[P]ₙ-Y-Z-Y'-[P']ₘ-NH-CH(R1 )-X'
wherein
X and X' are identical or different and are groups selected from aldehyde, boronic acid, boronic acid ester, α-ketoaldehyde, α-ketoester, α-ketoamide, trifluoromethylketone, diazomethane, isocoumarin derivatives, α',β'-epoxyketone, vinyl sulfone, chloromethyl and diazomethyl ketone and sulfonyl-fluoride;
R1 and R1' are identical or different and correspond to the specificity of the S1 enzyme subsite for chymotrypsin- or trypsin-like or PGPH activity of the proteasome and are selected from hydrophobic side chains of L or D, natural and unnatural amino acids, branched and unbranched, saturated and unsaturated alkyl, cycloalkyl, alkaryl, aralkyl residues which may be amino-, guanido-, amidino- or carboxy-substituted;
P and P' are identical or different and correspond to the P2 to P7 substrate positions and are natural or unnatural L or D amino acids with hydrophobic or hydrophylic or charged side chains;
m and n are identical or different and are 0, 1, 2, 3, 4, 5 or 6;
Y and Y' are identical or different and correspond to the formula CO-(A)-(B) wherein (A) is an alkyl, alkenyl, cycloalkyl, alkaryl, aralkyl or heteroaromatic group and (B) is CO or NH or omitted; and
Z is O-CH₂-CH₂-(O-CH₂-CH₂)ₙ-O, OC-CH₂-(O-CH₂-CH₂)ₙ₋₁-O-CH₂-CO or NH-CH₂-CH₂-(O-CH₂-CH₂)ₙ-NH with n varying from 4 to 50.

4. The bivalent inhibitor compound according to one of the previous claims,
wherein the said side chains are linear or branched alkyl, cycloalkyl, alkenyl, aryl, and -CH₂-R2 where R2 is aryl, aralkyl, alkaryl, cycloalkyl or -Q-R3 where Q is a chalcogen, and R3 is alkyl.

5. The bivalent inhibitor compound according to one of the previous claims,
wherein the alkyl and alkenyl groups per se or within aralkyl or alkaryl residues each contain from 1 to 20 resp. 2 to 20 C atoms.

6. The bivalent inhibitor compound according to claim 5 wherein the alkyl and alkenyl groups each contain 1 to 12 resp. 2 to 12 C atoms.

7. The bivalent inhibitor compound according to claim 6 wherein alkyl and alkenyl groups bound to an aryl residue contain 1 to 8 resp. 2 to 8 C atoms.

8. The bivalent inhibitor compound according to one of the previous claims,
wherein it is a homobivalent inhibitor.

9. The bivalent inhibitor compound according to claim 8,
wherein it is (Polyoxyethylene)₁₉_{*-*}₂₅-[NH-CO-(CH₂)₂-CO-Leu-Leu-Nle-H]₂.

10. The bivalent inhibitor compound according to one of the previous claims 1 to 7,
wherein it is a heterobivalent inhibitor.

11. Method for preparing a bivalent proteasome inhbitor compound comprising linking two monovalent proteasome inhibitor molecules capable of inhibiting one or more active sites of the proteasome with a spacer comprising a flexible, substantially linear organic compound with a length of 25 Å to 100 Å.

12. The method according to claim 11, wherein the two monovalent protease inhibitor molecules are linked with the terminal amino or carboxy groups of a polyoxyethylene derivative containing 4 to 50 oxyethylene units.

13. The method according to claim 12 wherein a polyoxyethylene derivative containing 18 to 25 oxyathylene units is used.

14. The method according to claim 13 wherein a polyoxyethylene derivative containing 19 to 25 oxyethylene units is used.

15. A pharmaceutical composition for the inhibition of proteasome function, comprising a bivalent proteasome inhibitor according to one of claims 1 to 10 as effective compound or a physiologically acceptable salt thereof, and optionally physiological carriers, additives, thinners and/or auxiliary substances.

16. Use of bivalent inhibitor compound according to one of claims 1 to 10 or a pharmaceutical composition according to claim 15 for reducing the rate of intracellular protein breakdown.

17. Use of bivalent inhibitor compound according to one of claims 1 to 10 or a pharmaceutical composition according to claim 15 for inhibiting the activity of NF-κB in a cell.

18. Use of bivalent inhibitor compound according to one of claims 1 to 10 or a pharmaceutical composition according to claim 15 for reducing the rate of degradation of p53 protein in a cell.

19. Use of bivalent inhibitor compound according to one of claims 1 to 10 or a pharmaceutical composition according to claim 15 for inhibiting cyclin degradation in a cell.

20. Use of bivalent inhibitor compound according to one of claims 1 to 10 or a pharmaceutical composition according to claim 15 for inhibiting inflammatory responses in a mammal.

21. Use of bivalent inhibitor compound according to one of claims 1 to 10 or a pharmaceutical composition according to claim 15 for inhibiting antigen presentation in a cell.

22. Use of bivalent inhibitor compound according to one of claims 1 to 10 or a pharmaceutical composition according to claim 15 for inhibiting inducible NF-κB dependent cell adhesion.

23. Use of bivalent inhibitor compound according to one of claims 1 to 10 or a pharmaceutical composition according to claim 15 for inhibiting HIV replication in a mammal.

24. Use of bivalent inhibitor compound according to one of claims 1 to 10 or a pharmaceutical composition according to claim 15 for reducing the rate of muscle protein degradation.

25. Use of bivalent inhibitor compound according to one of claims 1 to 10 or a pharmaceutical composition according to claim 15 for inhibiting the growth of a cancer cell.
